# EUROPEAN PATENT APPLICATION

(11) **EP 3 778 909 A1**
(43) Date of publication of application: **17.02.2021**
(21) Application number: 19775835.2
(22) Date of filing: 22.03.2019
(51) Int. Cl.: C12P 7/06, A01N 25/00, A01N 25/02, A01N 31/02, A01P 3/00, C12N 1/20

(54) **METHOD FOR PRODUCING ETHANOL AND ETHANOL COMPOSITION**

(30) Priority: 27.03.2018 JP 2018060699; 27.03.2018 JP 2018060746; 30.03.2018 JP 2018069742
(71) Applicant: SEKISUI CHEMICAL CO., LTD., Osaka-shi Osaka 530-8565 (JP)
(72) Inventor: NISHIYAMA Norihide, Tsukuba-shi, Ibaraki 300-4247 (JP); SATO Kanetomo, Tsukuba-shi, Ibaraki 300-4247 (JP); HAMACHI Kokoro, Tsukuba-shi, Ibaraki 300-4247 (JP); NATSUYAMA Kazuto, Tsukuba-shi, Ibaraki 300-4247 (JP)
(74) Representative: Ter Meer Steinmeister & Partner
(86) International application number: PCT/JP2019/012238
(87) International publication number: WO 2019/188839

(57) **Abstract**

This invention is intended to provide a production method to reduce sterilizability to bacteria and improve ethanol productivity and an ethanol composition with reduced sterilizability to bacteria. In a method of producing ethanol by fermenting a carbon source by a microorganism, a 2-propanol concentration in a microorganism fermenter is less than 2 ppm.

## Description

### Cross-Reference to Related Application

This application is based upon priority of the prior Japanese Patent Application No. 2018-60699, filed in Japan Patent Office on March 27, 2018, the prior Japanese Patent Application No. 2018-60746, filed in Japan Patent Office on March 27, 2018, the prior Japanese Patent Application No. 2018-69742, filed in Japan Patent Office on March 30, 2018, the entire contents of which are incorporated herein by reference.

### Technical Field

The present invention relates to a method of producing ethanol and an ethanol composition.

### Background Art

In recent years, from the perspective of measures to prevent global warming and effective use of wastes, use of biomass, the material which is plant resources, has become a focus of attention. As a general material to produce a compound such as ethanol from the biomass, saccharides, such as sugarcane, and starches, such as corn, are often used. However, these materials are originally used as food or feed, and thus long-term use as resources for industrial use has a possibility of causing conflict with food or feed applications and leading to a surge in material prices.

Technical development is thus pursued to use inedible biomass resources, such as construction scrap wood and agricultural wastes, as energy resources. Examples of such inedible biomass include cellulose, the most abundantly available on earth. Most cellulose is present as lignocellulose, which is a complex produced with lignin, being an aromatic polymer, and hemicellulose.

In ethanol production using lignocellulose-containing biomass, sugar acquired by pretreatment or enzymatic saccharification treatment is generally anaerobically fermented by yeast into ethanol. While a large amount of xylose is acquired from such lignocellulose-containing biomass, wild yeast is not capable of growing on xylose. Accordingly, as described in JP 2014-193152 A, it is investigated to use yeast imparted with capability to grow on xylose by gene manipulation.

However, due to legal restrictions at the national level in accordance with the Cartagena Protocol, use of the yeast imparted with capability to grow on xylose by gene manipulation takes heavy investments, such as acquisition of various types of environmental data and construction of closed system facilities. Particularly in Japan, the above problem is clear and thus it is difficult to use genetically modified yeast from the perspective of costs for production of an inexpensive substance, such as ethanol.

In recent years, as described in JP 2010-500026 A and JP 2012-526552 A, eubacteria, such as Escherichia coli, provided with capability to grow on both xylose and glucose have thus become a focus of attention. Since Escherichia coli is poor in resistance to self-generated ethanol compared with yeast, there is a problem of not being allowed to increase the ethanol concentration of a microbial fermentation broth, resulting in low productivity compared with the yeast method.

Continuous cropping of solanum, cucurbit, legumes, crucifers, and the like in farm lands, horticultural facilities, kitchen gardens, and the like is known to increase plant pathogenic bacteria and nematodes in the soil, often causing poor growth and blight. Such soil sickness due to continuous cropping is known to be solved by crop rotation, whereas continuous cropping is sometimes inevitable for reasons such as a business plan, a land area, and the like. In these cases, use of grafted nursery plants, disinfection with chemicals, and the like are performed.

However, the use of grafted nursery plants to cope with soil sickness due to continuous cropping leads to an increase in costs, and in addition, does not give a direct solution and is not capable of avoiding the soil sickness all the time over years. Major methods of disinfection with chemicals include soil sterilization by soil fumigation and various pesticides are distributed. However, these chemicals are assumed to have a problem of soil persistence and harmfulness to the human body and have a risk that the expression of organic agriculture becomes not available.

In recent years, as a method to obtain effects similar to pesticides using no pesticide and not affecting the human body, soil reduction sterilization methods using a low concentration aqueous ethanol solution have become a focus of attention as described in JP 2010-106034 A and WO 2007/129467 A1. In such a method, low concentration ethanol is supplied to the soil to grow soil microorganisms including bacteria growing on ethanol, and thus oxygen in the soil is consumed to be in an oxygen-free (reduced) state. In the method, as a result, pathogenic soil microorganisms and the like decrease or die out.

### Summary of Invention

### Technical Problem

Use of a eubacterium or an archaebacterium for fermentation by a microorganism causes the bacterium to die out due to the self-generated ethanol, and thus there is a problem of not allowing improvement in ethanol concentration in the microorganism fermenter.

A soil reduction sterilization method using a low concentration aqueous ethanol solution has a problem that, under the condition of using only solar heat, the soil has to be covered with a film and kept for as long as two to three weeks after ethanol distribution. Although early growing of soil microorganisms including bacteria growing on ethanol (hereinafter, referred to only as microorganisms) is assumed to reduce the time, an increase in ethanol concentration of the aqueous ethanol solution causes a new problem of sterilizing the soil microorganisms due to the sterilizability (bacteriostasis) of ethanol.

It is thus an object of the present invention to provide a production method to reduce sterilizability to bacteria and improve ethanol production and an ethanol composition with reduced sterilizability to bacteria.

### Solution to Problem

The present inventors made intensive investigations to find, as a result, that control of a 2-propanol concentration in a microorganism fermenter to a specific amount or less allows an increase in ethanol concentration of a microbial fermentation broth. An ethanol composition of the present invention has reduced sterilizability at a low concentration.

That is, the present invention is summarized as follows.
[1] A method of producing ethanol by fermenting a carbon source by a microorganism using a eubacterium or an archaebacterium, wherein a 2-propanol concentration in a microorganism fermenter is less than 2 ppm.
[2] The method of producing ethanol according to [1], wherein the carbon source contains a biomass resource.
[3] The method of producing ethanol according to [2], wherein the biomass resource is an inedible material.
[4] The method of producing ethanol according to [2] or [3], wherein the biomass resource is a waste.
[5] The method of producing ethanol according to any one of [1] through [4], wherein the microorganism is a eubacterium.
[6] The method of producing ethanol according to any one of [1] through [5], wherein an ethanol concentration in the microorganism fermenter is 0.1 mass% or more and 15 mass% or less.
[7] Ethanol produced by the method according to any one of [1] through [6].
[8] An ethanol composition having a 2-propanol concentration of less than 2 ppm.
[9] An ethanol composition including 0.001 ppm or more and 1000 ppm or less of a saturated hydrocarbon compound with a carbon number from 6 to 14.
[10] The ethanol composition according to [9], comprising 50 mass% or more of ethanol.
[11] The ethanol composition according to any one of [8] through [10], further including from 0.001 ppm to 800 ppm of an aromatic hydrocarbon compound with a carbon number from 6 to 14.
[12] The ethanol composition according to any one of [8] through [11], wherein the saturated hydrocarbon compound is one or more selected from the group consisting of heptane, octane, and decane.
[13] An ethanol composition including from 0.001 ppm to 100 ppm of an aromatic compound with a carbon number from 6 to 14.
[14] The ethanol composition according to Claim [13], wherein the aromatic compound is one or more selected from the group consisting of toluene, benzene, ethylbenzene, xylene, and naphthalene.
[15] The ethanol composition according to [8], [13], or [14], further including from 0.001 ppm to 1000 ppm of a saturated hydrocarbon compound with a carbon number from 6 to 14.
[16] The ethanol composition according to any one of [8] through [15], including 50 mass% or more of ethanol.
[17] An ethanol composition including from 0.001 ppm to 1000 ppm of n-decane.
[18] The ethanol composition according to [17], including from 0.001 ppm to 800 ppm of ethylbenzene.
[19] An ethanol composition including from 0.001 ppm to 100 ppm of ethylbenzene.
[20] The ethanol composition according to [19], including from 0.001 ppm to 1000 ppm of n-decane.
[21] A soil disinfection method, including:
   diluting the ethanol composition according to any one of [8] through [20] with water; and
   distributing an aqueous ethanol solution with an ethanol concentration after dilution from 0.1 to 15 mass %.

### Advantageous Effects of Invention

The method of producing ethanol of present invention allows an increase in the upper limit of ethanol concentration in the microorganism fermenter, and it is thus possible to increase an amount of production per unit time and to reduce distillation costs in a later procedure.

The above effects of the present invention are similarly expected even for a genetically modified eubacterium for ethanol production.

The ethanol composition of the present invention has low sterilizability under the condition of low concentration approximately from 1 to 15 mass%. It is thus possible to be preferably used as a solvent for cosmetics applied to the skin, ethanol for soil reduction sterilization, and the like.

### Description of Embodiments

Preferred modes for carrying out the present invention are described below with examples. It should be noted that the following embodiments are examples to describe the present invention and the present invention is not at all limited to the following embodiments. In the present invention, ppm means mass ppm unless otherwise specified.

### Method of Producing Ethanol

### Microorganism

A microorganism that may be used in the method of the present invention is not particularly limited as long as it is a eubacterium or an archaebacterium provided with ethanol productivity, and examples of the microorganism generally include: eubacteria, such as Zymomonas, Escherichia coli, Corynebacterium, Clostridium, and Halomonas; and archaebacteria, such as Halobacteria. From the perspective of ethanol productivity, the eubacteria of Zymomonas, Escherichia coli, Corynebacterium, Clostridium, and Halomonas are preferably used, and Escherichia coli and Clostridium are more preferably used.

More specific examples of the eubacterium include Zymomonas mobilis, genetically modified Escherichia coli (strain KO11), Clostridium Ijungdahlii, Clostridium autoethanogenum, and Halomonas sp. KM-1, and they are preferably used.

### Carbon Source

In the method of the present invention, a carbon source used as a material for fermentation by a microorganism is not particularly limited, as long as the material contains carbon, and preferably contains a biomass resource. The biomass resource may be either an edible material or an inedible material, and from the perspective of food resource conservation, an inedible material is preferred and an inedible waste material is more preferred. Specific examples of the material to be used include: edible materials, such as sugarcane and corn; woody biomass materials, such as chips and bark of papermaking wood, thinnings left in the forest, thinnings, and the like, sprouts originating from stumps of woody plants, sawmill waste and sawdust from sawmills, pruned branches and leaves of roadside trees, and construction scrap wood; grass biomass materials, such as kenaf, rice straw, straw, corncobs, and bagasse; agricultural wastes; residues of industrial crops, such as oil crops and rubber; paper derived from wood; wastepaper; pulp; pulp sludge; sludge; sewage sludge; food wastes; and the like. Among all, it is more preferred to contain any of woody biomass materials, agricultural wastes, sewage sludge, and food wastes.

The carbon source used in the method of the present invention includes, for example, the biomass resources described above subjected to pretreatment, such as mechanical treatment including cutting, chopping, crushing, grinding, and the like, chemical treatment using acid, alkali, and various chemicals, heat treatment including heating, fumigation, and the like, saccharification treatment by addition of enzymes or other microorganisms, and gasification treatment by incomplete combustion (production of CO).

### Mechanical Treatment

Examples of the mechanical treatment include arbitrary mechanical mechanisms, such as cutting, chopping, crushing, and grinding, of the biomass resource to cause the biomass resource to be in a state facilitating saccharification and fermentation. Examples of the mechanical device to be used include, but not particularly limited to, single-roll crushers, double-roll crushers, hammer crushers, refiners, kneaders, ball mills, and the like.

### Chemical Treatment

For chemical treatment, the biomass resource may be immersed in a solution containing: one or more alkaline chemicals selected from sodium hydroxide, potassium hydroxide, calcium hydroxide, sodium carbonate, and sodium hydrogen carbonate; or one or more alkaline chemicals selected from sodium sulfite and the above alkaline chemicals. The contact temperature and time may be appropriately set in accordance with the intended biomass resource.

### Gasification Treatment

The gasification treatment is a procedure to generate source gas containing carbon monoxide by gasifying the biomass resource in a gasification section. The gasification section is a furnace for combustion (incomplete combustion) of the carbon source, and examples of the gasification section include gasification furnaces, such as a shaft furnace, a kiln furnace, a fluidized bed furnace, and a gasification reformer furnace.

### Saccharification Treatment

The saccharification treatment may be carried out by mixing the biomass resource with an appropriate amount of water and an enzyme and then setting at an appropriate temperature. More specifically, addition of cellulase or hemicellulose as the enzyme allows saccharification of the biomass resource (e.g., cellulose to glucose, hemicellulose to glucose and xylose).

### Procedure of Fermentation by Microorganism

The procedure of fermentation by a microorganism is a procedure to produce ethanol by fermenting the above carbon source by a microorganism using a eubacterium or an archaebacterium in a microorganism fermenter. The microorganism fermenter is preferably a continuous fermentation device. The microorganism fermenter to be used may be generally in an arbitrary shape, and examples of the microorganism fermenter include a stirring fermenter, an air lift fermenter, a bubble tower fermenter, a loop fermenter, an open bonding fermenter, and a photobiofermenter.

The ethanol concentration in the microorganism fermenter may be appropriately adjusted in accordance with the properties of the microorganism. The ethanol concentration is generally 0.01 mass% or more, preferably 0.1 mass% or more, more preferably 1 mass% or more, and even more preferably 3 mass% or more. Meanwhile, the upper limit of the ethanol concentration is generally 15 mass% or less, preferably 12 mass% or less, more preferably 10 mass% or less, and even more preferably 8 mass% or less. A low ethanol concentration tends to cause an increase in purification costs in a later purification procedure, whereas a too high ethanol concentration tends to cause a decrease in viability of the microorganism due to the sterilizability of ethanol. The ethanol concentration in the microorganism fermenter in the present invention means the ethanol concentration at the time of being discharged from the microorganism fermenter.

In the present invention, the carbon source and a microorganism culture broth are continuously supplied to the microorganism fermenter while the carbon source and the microorganism culture broth do not have to be simultaneously supplied and the carbon source may be supplied to the microorganism fermenter where the microorganism culture broth is supplied in advance. When the saccharification treatment is carried out concurrently with fermentation by a microorganism, the above enzyme may be contained in a culture medium.

In the present invention, a 2-propanol concentration in the microorganism fermenter is less than 2 ppm. The 2-propanol concentration in the microorganism fermenter is preferably 1.8 ppm or less, preferably 1.5 ppm or less, more preferably 1.2 ppm or less, and even more preferably 1 ppm or less. Meanwhile, the lower limit of the 2-propanol concentration is generally, but not particularly limited to, 0.0001 ppm or more, preferably 0.001 ppm or more, more preferably 0.01 ppm or more, and even more preferably 0.1 ppm or more. A too high 2-propanol concentration in the microorganism fermenter causes the microorganism to be sterilized by self-generated ethanol and die out. A too low 2-propanol concentration causes an increase in removal costs and deterioration in productivity. In the present invention, to have the 2-propanol concentration less than 2 ppm means to control the 2-propanol concentration in the above range for 50% or more time in the entire culture period.

The reason why the ethanol resistance of the microorganism is improved by the 2-propanol concentration in the microorganism fermenter of less than 2 ppm in the present invention is not certain but assumed as follows.

It is conventionally known that a volatile organic compound (VOC) is contained in woody and grass materials, particularly woody construction scrap wood and 2-propanol or the like is generally contained as the VOC component (JIS1964: 2015 appendix A1, etc.). Among all, 2-propanol has properties, such as a boiling point, similar to ethanol and is difficult to be isolated by general distillation or the like. Even after general pretreatment, 2-propanol contained in the biomass resource is thus contained in a trace amount in the microorganism fermenter and a resulting product.

It is then considered that such a minor component does not affect sterilizability (bacteriostasis) at all in purified ethanol with the concentration generally used for sterilization while greatly affecting sterilizability to microorganisms in the presence of low concentration ethanol approximately from 1 to 15 mass%.

That is, the sterilization action to microorganisms by low concentration ethanol is considered to inhibit an outflow of an intracellular matrix and an uptake of nutrients by disturbing the cell membrane, and thus 2-propanol with greater degreasing power than ethanol contained in the culture broth is assumed to cause an increase in the sterilization effect.

In the present invention, it is considered that control of the amount of 2-propanol in the materials at the stage of pretreatment or a culture procedure allows inhibition of sterilizability of ethanol more than the past and production of ethanol with higher productivity.

The ethanol composition produced in the method of the present invention is considered to have reduced sterilizability for reasons similar to above when used with an ethanol concentration from 1 to 15 mass%.

In the present invention, a method of controlling 2-propanol in the microorganism fermenter is not particularly limited as long as it is a known mechanism, and for example, the method may only use materials containing less 2-propanol in the materials or include: a procedure, at an arbitrary stage from materials to the procedure of fermentation by a microorganism, to contact with an ion exchange resin, active carbon, zeolite, or the like provided with 2-propanol removal performance; a drying procedure; a procedure to produce a solution by saccharification treatment or the like and then separate by distillation; and a membrane separation procedure using various types of dendrimer membrane, zeolite membrane, and the like.

### Purification Procedure

In the method of producing ethanol according to the present invention, an ethanol-containing liquid obtained by fermentation by a microorganism may be made available for the later purification procedure for the purpose of purification. The purification procedure is a procedure to separate, in the purification section, the ethanol-containing liquid obtained in the fermentation procedure into a distillate with an increased intended ethanol concentration and a residual liquid with a decreased intended ethanol concentration. Examples of the device used in the purification procedure include a distillation device, a treatment device containing a pervaporation membrane, a treatment device containing a zeolite dehydration membrane, a treatment device to remove low boiling substances having a boiling point lower than ethanol, a treatment device to remove high boiling substances having a boiling point higher than ethanol, a treatment device containing an ion exchange membrane, and the like. Any of the devices may be used singly or two or more types of them may be used in combination. For unit operation, heating distillation and membrane separation may be preferably used.

For distillation, it is possible to use such a distillation device. The temperature in the still during distillation of ethanol is preferably, but not particularly limited to, 100°C or less and more preferably from 70°C to 95°C approximately. Setting of the temperature in the still in the above range allows more secure separation of the desired ethanol from other components, that is, distillation (purification) of ethanol.

The pressure in the distillation device during distillation of ethanol may be normal pressure and is preferably less than atmospheric pressure and more preferably from 60 to 95 kPa (gauge pressure) approximately. Setting of the pressure in the distillation device in the above range allows improvement in efficiency of ethanol separation, leading to improvement in the yield of ethanol.

For membrane separation, it is possible to appropriately use a known separation membrane, and for example, a zeolite membrane may be preferably used.

The concentration of ethanol contained in the distillate separated in the purification procedure is preferably from 20 mass% to 99.9 mass% and more preferably from 60 mass% to 99.99 mass%. Meanwhile, the concentration of ethanol contained in the residual liquid is preferably from 0.001 mass% to 10 mass% and more preferably from 0.01 mass% to 5 mass%.

It is possible to reduce 2-isopropanol in purified ethanol by appropriately setting the temperature, the pressure, and the separation membrane during distillation as described above.

### Ethanol Composition

According to the first embodiment of the present invention, the 2-propanol concentration in the ethanol composition is less than 2 ppm. The 2-propanol concentration in the ethanol composition is preferably 1.8 ppm or less, preferably 1.5 ppm or less, more preferably 1.2 ppm or less, and even more preferably 1 ppm or less. Meanwhile, the lower limit is generally 0 ppm or more, preferably 0.001 ppm or more, and more preferably 0.01 ppm or more. The2-propanol content in the ethanol composition in the above range causes reduction in sterilizability of the ethanol composition in use at a low concentration.

The ethanol concentration in the ethanol composition of the present invention is, but not particularly limited to, generally 1 mass% or more based on the total amount of the ethanol composition, preferably 3 mass% or more, more preferably 10 mass% or more, even more preferably 50 mass% or more, particularly preferably 90 mass% or more, and most preferably 98 mass% or more. Meanwhile, the upper limit is generally 99.999 mass% or less, preferably 99% or less, more preferably 90 mass% or less, even more preferably 80 mass% or less, particularly preferably 60 mass% or less, and most preferably 15 mass% or less. The ethanol concentration in the ethanol composition of the present invention may be set in accordance with the intended application, and is preferably 75 mass% or more for ethanol for disinfection and 50 mass% or more for ethanol for soil disinfection. The upper limit conveniently set in the similar manner in accordance with the application. From the perspective of transportation costs, ethanol is generally distributed and sold in the form of an ethanol composition of 95% or more and often diluted with water or a solvent in accordance with the target concentration.

The ethanol composition of the present invention may contain an aromatic compound with a carbon numberfrom 6 to 14. Examples of the aromatic compound with a carbon number from 6 to 14 include: monocyclic aromatic compounds, such as benzene, toluene, xylene, mesitylene, cumene, ethylbenzene, butylbenzene, styrene, α-methylstyrene, o-methylstyrene, m-methylstyrene, p-methylstyrene, vinylxylene, ptert-butylstyrene, and ethylstyrene; and polycyclic aromatic compounds, such as naphthalene, phenanthrene, anthracene, and pyrene. The compound preferably has a carbon number from 6 to 10 and more preferably has a carbon number from 6 to 8, and among all, a preferred compound is a monocyclic aromatic compound containing one or more alkyl chains with a carbon number from 1 to 4 and a more preferred compound is a monocyclic aromatic compound containing one alkyl chain with a carbon number from 1 to 4. More specifically, it is even more preferred to contain any of benzene, toluene, xylene, and ethylbenzene, and particularly preferred to contain toluene.

When the aromatic compound is contained in the ethanol composition, the amount is 0.001 ppm or more of the sum total of the aromatic compound in the ethanol composition, preferably 0.005 ppm or more, more preferably 0.01 ppm or more, and even more preferably 0.1 ppm% or more. Meanwhile, the upper limit is 100 ppm or less, preferably 80 ppm or less, more preferably 50 ppm or less, even more preferably 30 ppm or less, particularly preferably 20 ppm or less, most preferably 10 ppm or less, and particularly preferably 1 ppm or less. The aromatic compound contained in the ethanol composition in the above range tends to cause reduction in sterilizability of the ethanol composition.

The ethanol composition of the present invention may contain a saturated hydrocarbon compound with a carbon number from 6 to 14.

When the saturated hydrocarbon compound with a carbon number from 6 to 14 is contained in the ethanol composition of the present invention, examples of the saturated hydrocarbon compound include hexane, heptane, octane, nonane, decane, undecane, dodecane, tridecane, and tetradecane. Among all, the saturated hydrocarbon compound is preferably a saturated hydrocarbon compound with a carbon number from 7 to 11, more preferably a saturated hydrocarbon compound with a carbon number from 7 to 10, more preferably heptane, octane, and decane, and most preferably n-decane with a carbon number of 10. The saturated hydrocarbon compound with a carbon number in the above range tends to cause inhibition of sterilizability of ethanol.

When the ethanol composition contains the saturated hydrocarbon compound, the sum total of the saturated hydrocarbon compound in the ethanol composition is generally 0.001 ppm or more, preferably 0.005 ppm or more, more preferably 0.01 ppm or more, and even more preferably 0.1 mass% or more. Meanwhile, the upper limit is generally 100 ppm or less, preferably 80 ppm or less, more preferably 50 mass ppm or less, even more preferably 10 ppm or less, and particularly preferably 1 ppm or less. Saturated hydrocarbon contained in the ethanol composition in the above range tends to cause inhibition of sterilizability of the ethanol composition.

The content of the optional components contained in the ethanol composition may be adjusted by addition to purified ethanol.

According to the second embodiment of the present invention, the ethanol composition of the present invention contains from 0.001 ppm to 100 ppm of an aromatic compound with a carbon number from 6 to 14.

The ethanol concentration in the ethanol composition of the present invention is, but not particularly limited to, generally 40 mass% or more based on the total amount of the ethanol composition, preferably 50 mass% or more, more preferably 60 mass% or more, even more preferably 75 mass% or more, particularly preferably 90 mass% or more, and most preferably 98 mass% or more. Meanwhile, the upper limit is generally 99.999 mass% or less, preferably 99% or less, more preferably 90 mass% or less, even more preferably 80 mass% or less, and particularly preferably less than 60 mass%. The ethanol concentration in the ethanol composition of the present invention may be set in accordance with the intended application, and is preferably 90 mass% or more for a solvent for cosmetics and the like, 75 mass% or more for ethanol for disinfection, and 50 mass% or more for ethanol for soil disinfection. The upper limit may be conveniently set in the similar manner in accordance with the application. As an embodiment of the ethanol composition of the present invention, the ethanol composition is often used by being diluted with water or a solvent, whereas from the perspective of transportation costs and the like, a higher concentration of the ethanol composition is preferred as products.

It should be noted that, for sale as ethanol for soil disinfection through a retail store, such as a home center, or temporary storage by a user, the ethanol concentration of less than 60 mass% is preferred for not requiring special management as a hazardous material under the provisions of the Fire Service Act in Japan. Existing materials for soil reduction disinfection are sold under the trade name of ECOLOGA^{∼}L® from Japan Alcohol Corp. and the like.

The ethanol composition of the present invention contains an aromatic compound with a carbon number from 6 to 14. Examples of the aromatic compound with a carbon number from 6 to 14 include: monocyclic aromatic compounds, such as benzene, toluene, xylene, mesitylene, cumene, ethylbenzene, butylbenzene, styrene, α-methylstyrene, o-methylstyrene, m-methylstyrene, p-methylstyrene, vinylxylene, ptert-butylstyrene, and ethylstyrene; and polycyclic aromatic compounds, such as naphthalene, phenanthrene, anthracene, and pyrene. From the perspective of soil degradability, the aromatic compound preferably has a carbon number from 6 to 10 and more preferably has a carbon number from 6 to 8, and among all, a preferred compound is a monocyclic aromatic compound containing one or more alkyl chains with a carbon number from 1 to 4 and a more preferred compound is a monocyclic aromatic compound containing one alkyl chain with a carbon number from 1 to 4. More specifically, it is even more preferred to contain any of benzene, toluene, xylene, and ethylbenzene, and particularly preferred to contain ethylbenzene.

The amount of the aromatic compound contained in the ethanol composition is 0.001 ppm or more of the sum total of the aromatic compound in the ethanol composition, preferably 0.005 ppm or more, more preferably 0.01 ppm or more, and even more preferably 0.1 ppm% or more. Meanwhile, the upper limit is 100 ppm or less, preferably 80 ppm or less, more preferably 50 ppm or less, even more preferably 30 ppm or less, particularly preferably 20 ppm or less, and most preferably 10 ppm or less. Such aromatic compounds often include cytotoxic compounds and thus a too much content causes a decrease in viability of the microorganism and also causes soil contamination. A too little content of the aromatic compound causes failure to obtain the effect of reducing sterilizability to the microorganisms.

When the ethanol composition contains ethylbenzene, 0.001 ppm or more of ethylbenzene is contained, preferably 0.005 ppm or more, more preferably 0.01 ppm or more, and even more preferably 0.1 mass% or more. Meanwhile, the upper limit is 100 ppm or less, preferably 80 ppm or less, more preferably 50 mass ppm or less, even more preferably 30 ppm or less, particularly preferably 10 ppm or less, and most preferably 1 ppm or less. Ethylbenzene is concerned to be cytotoxic, and thus a too much ethylbenzene content causes a risk of dying out of the bacteria and a risk of soil contamination and a too little content is concerned to cause failure to obtain the effects of the present invention.

The ethanol composition according to the second embodiment of the present invention may further contain a saturated hydrocarbon compound with a carbon number from 6 to 14 as a constituent other than the aromatic compound with a carbon number from 6 to 14. Examples of the saturated hydrocarbon compound with a carbon number from 6 to 14 include hexane, heptane, octane, nonane, decane, undecane, dodecane, tridecane, and tetradecane. Among all, the saturated hydrocarbon compound is preferably a saturated hydrocarbon compound with a carbon number from 7 to 11, more preferably a saturated hydrocarbon compound with a carbon number from 7 to 10, more preferably heptane, octane, and decane, and most preferably n-decane with a carbon number of 10. The saturated hydrocarbon compound with a carbon number in the above range tends to cause inhibition of sterilizability of ethanol.

When the ethanol composition contains the saturated hydrocarbon compound, the sum total of the saturated hydrocarbon compound is generally 0.001 ppm or more, preferably 0.005 ppm or more, more preferably 0.01 ppm or more, and even more preferably 0.1 mass% or more. Meanwhile, the upper limit is generally 1000 ppm or less, preferably 800 ppm or less, more preferably 500 mass ppm or less, even more preferably 100 ppm or less, particularly preferably 10 ppm or less, and most preferably 1 ppm or less. A too much content of the saturated hydrocarbon compound causes a risk of soil contamination and a too little content is concerned to cause failure to obtain the effects of the present invention.

When the ethanol composition contains n-decane, 0.001 ppm or more of n-decane is generally contained, preferably 0.005 ppm or more, more preferably 0.01 ppm or more, and even more preferably 0.1 mass% or more. Meanwhile, the upper limit is generally 1000 ppm or less, preferably 800 ppm or less, more preferably 500 mass ppm or less, even more preferably 100 ppm or less, particularly preferably 10 ppm or less, and most preferably 1 ppm or less. A too much content of n-decane causes a risk of soil contamination and a too little content is concerned to cause failure to obtain the effects of the present invention.

According to the third embodiment of the present invention, the ethanol composition of the present invention contains from 0.001 ppm to 1000 ppm of a saturated hydrocarbon compound with a carbon number from 6 to 14.

The ethanol concentration in the ethanol composition of the present invention is, but not particularly limited to, generally 40 mass% or more based on the total amount of the ethanol composition, preferably 50 mass% or more, more preferably 60 mass% or more, even more preferably 75 mass% or more, particularly preferably 90 mass% or more, and most preferably 98 mass% or more. Meanwhile, the upper limit is generally 99.999 mass% or less, preferably 99% or less, more preferably 90 mass% or less, even more preferably 80 mass% or less, and particularly preferably less than 60 mass%. The ethanol concentration in the ethanol composition of the present invention may be set in accordance with the intended application, and is preferably 90 mass% or more for a solvent for cosmetics and the like, 75 mass% or more for ethanol for disinfection, and 50 mass% or more for ethanol for soil disinfection. The upper limit may be conveniently set in the similar manner in accordance with the application. As an embodiment of the ethanol composition of the present invention, the ethanol composition is often used by being diluted with water or a solvent, whereas from the perspective of transportation costs and the like, a higher concentration of the ethanol composition is preferred as products.

It should be noted that, for sale as ethanol for soil disinfection through a retail store, such as a home center, or temporary storage by a user, the ethanol concentration of less than 60 mass% is preferred for not requiring special management as a hazardous material under the provisions of the Fire Service Act in Japan. Existing materials for soil reduction disinfection are sold under the trade name of ECOLOGA^{∼}L® from Japan Alcohol Corp. and the like.

The ethanol composition of the present invention contains a saturated hydrocarbon compound with a carbon number from 6 to 14. Examples of the saturated hydrocarbon compound include hexane, heptane, octane, nonane, decane, undecane, dodecane, tridecane, and tetradecane. Among all, the saturated hydrocarbon compound is preferably a saturated hydrocarbon compound with a carbon number from 7 to 11, more preferably a saturated hydrocarbon compound with a carbon number from 7 to 10, more preferably heptane, octane, and decane, and most preferably n-decane with a carbon number of 10. The saturated hydrocarbon compound with a carbon number in the above range tends to cause inhibition of sterilizability of ethanol.

In the ethanol composition of the present invention, 0.001 ppm or more of the sum total of the saturated hydrocarbon compound is contained in the ethanol composition, preferably 0.005 ppm or more, more preferably 0.01 ppm or more, and even more preferably 0.1 ppm% or more. Meanwhile, the upper limit is 1000 ppm or less, preferably 800 ppm or less, more preferably 500 mass ppm or less, even more preferably 100 ppm or less, particularly preferably 10 ppm or less, and most preferably 1 ppm or less. A too much content of the saturated hydrocarbon compound causes a decrease in viability of the microorganism and also causes soil contamination. A too little content of the saturated hydrocarbon compound causes failure to obtain the effect of reducing sterilizability to the microorganisms.

When the ethanol composition contains n-decane, the content is generally 0.001 ppm or more, preferably 0.005 ppm or more, more preferably 0.010 ppm or more, and even more preferably 0.1 mass% or more. Meanwhile, the upper limit is generally 1000 ppm or less, preferably 800 ppm or less, more preferably 500 mass ppm or less, even more preferably 100 ppm or less, particularly preferably 10 ppm or less, and most preferably 1 ppm or less. A too much content of n-decane causes a risk of soil contamination and a too little content causes failure to obtain the effect of reducing sterilizability to the microorganisms.

The ethanol composition according to the third embodiment of the present invention may further contain an aromatic compound with a carbon number from 6 to 14 as a constituent other than the saturated hydrocarbon compound with a carbon number from 6 to 14.

Examples of the aromatic compound with a carbon number from 6 to 14 include: monocyclic aromatic compounds, such as benzene, toluene, xylene, mesitylene, cumene, ethylbenzene, butylbenzene, styrene, α-methylstyrene, o-methylstyrene, m-methylstyrene, p-methylstyrene, vinylxylene, ptert-butylstyrene, and ethylstyrene; and polycyclic aromatic compounds, such as naphthalene, phenanthrene, anthracene, and pyrene. From the perspective of soil degradability, the aromatic compound preferably has a carbon number from 6 to 10 and more preferably has a carbon number from 6 to 8, and among all, a preferred compound is a monocyclic aromatic compound containing one or more alkyl chains with a carbon number from 1 to 4 and a more preferred compound is a monocyclic aromatic compound containing one alkyl chain with a carbon number from 1 to 4. More specifically, it is even more preferred to contain any of benzene, toluene, xylene, and ethylbenzene, and particularly preferred to contain ethylbenzene.

When the ethanol composition contains the aromatic compound, the content is generally 0.001 ppm or more of the sum total of the aromatic compound in the ethanol composition, preferably 0.005 ppm or more, more preferably 0.01 ppm or more, and even more preferably 0.1 ppm% or more. Meanwhile, the upper limit is generally 800 ppm or less, preferably 500 ppm or less, more preferably 100 ppm or less, even more preferably 50 ppm or less, particularly preferably 20 ppm or less, and most preferably 10 ppm or less.

Such aromatic compounds often include cytotoxic compounds and thus a too much content causes a decrease in viability of the microorganism and also causes soil contamination. A too little content causes failure to obtain the effect of reducing sterilizability to the microorganisms.

The ethanol composition of the present invention may contain ethylbenzene. The content in the ethanol composition in this case is generally 0.001 ppm or more, preferably 0.005 ppm or more, more preferably 0.010 ppm or more, and even more preferably 0.1 ppm% or more. Meanwhile, the upper limit is generally 800 ppm or less, preferably 500 ppm or less, more preferably 100 ppm or less, even more preferably 50 ppm or less, particularly preferably 20 ppm or less, and most preferably 10 ppm or less. Ethylbenzene is cytotoxic, and thus a too much ethylbenzene content causes a risk of dying out of the bacteria and a risk of soil contamination and a too little content is concerned to cause failure to obtain the effects of the present invention.

It is an object of the present invention to provide an ethanol composition having inhibited sterilizability (bacteriostasis) to microorganisms including the bacteria growing on ethanol in the soil and thus it is preferred to perform soil tests considering the above common sense in the technique. It is however difficult to perform soil tests under entirely the identical conditions and find a significant difference for the perspective of facilities, analysis techniques, and testing periods. The present invention employs substitution for such a soil test in which Escherichia coli is selected as a biological model for the eubacterium (bacterium) to evaluate the sterilizability. As a result, it is found that the ethanol composition containing a trace amount of the aromatic compound has alleviated sterilizability to the microorganisms compared with synthetic ethanol synthesized from petroleum and ethanol produced by sugar fermentation derived from a biomass resource.

A reason for this result is assumed to be that the sterilization action in low concentration ethanol with an ethanol concentration from 1 to 15 wt% causes ethanol to disturb the membrane order of cell membranes (phospholipid) and outflows of part of bacterial contents and uptakes of nutrition from outside the bacteria.

The aromatic compound with a carbon number from 6 to 14 defined in the present invention is highly hydrophobic and is thus considered to move from the area of the cell membrane disturbed by the low concentration ethanol (that is, the local area with low hydrophilicity) to a hydrophobic layer in the cell membrane and restore the disturbance in the cell membrane with the hydrophobicity to allow inhibition of the sterilizability of ethanol.

When the aromatic compound is benzene, benzene is considered to move (rotate) in the hydrophobic layer of the cell membrane to damage the cell membrane from inside. However, particularly in the case of a monocyclic aromatic compound containing an alkyl chain, for example, ethylbenzene, it is assumed to be more preferred because such a monocyclic aromatic compound is stabilized in a position parallel to the cell membrane and does not affect the cell membrane after the uptake.

### Applications

The ethanol composition of the present invention may be used as a soil sterilizer due to the low sterilizability to the microorganism and as a material for cosmetics, such as non-irritant perfume, and an ethanol derivative due to the less influence on indigenous skin bacteria.

When the ethanol composition of the present invention is used as a soil sterilizer, it is preferred to dilute a stock solution with water to have an ethanol concentration from 0.5% to 3% in the ethanol composition and distribute the diluted solution over the soil and then cover the soil with a film. The film is then kept for weeks to reduce oxygen in the soil and thus various aerobic bacteria and insects are removed. A more detailed method of use is disclosed in "Soil Disinfection Manual by Soil Reduction Action using Low Concentration Ethanol" by National Institute for Agro-Environmental Sciences, Japan.

### Examples

Although the present invention will be described in further detail with reference to Examples, the present invention is not limited to Examples below.

### Bacterial Strain, Reagents, Culture Mediums, and Devices

**[Table 1]**

| Bacterial Strain | | |
|---|---|---|
| Abbreviation | Name of Bacterial Strain | Type of Used Bacterium |
| *S. aureus* | *Staphylococcus aureus subsp. aureus* | NBRC 12732 |

**[Table 2]**

| Used Culture Mediums and Reagents | | |
|---|---|---|
| Abbreviation | Name of Culture Medium and Reagent | Name of Manufacturer |
| SCD | Nissui Plate Trypto-Soya Agar Culture Medium | Nissui Pharmaceutical Co., Ltd. |
| SCD-A | Trypcase Soy Agar Culture Medium | Sysmex bioMérieux Co., Ltd. |
| SCD-B | Trypcase Soy Broth | Sysmex bioMérieux Co., Ltd. |
| | SCDLP Broth Culture Medium | Eiken Chemical Co., Ltd. |
| PW | Purified Water in Japanese Pharmacopoeia | Yamazen Corp. |
| SAL | Otsuka Normal Saline in Japanese Pharmacopoeia | Otsuka Pharmaceutical Co., Ltd. |

**[Table 3]**

| Used Tools | |
|---|---|
| Tool Name | Name of Manufacturer |
| 15 mL Centrifuge Tubes | Corning Japan K.K. |
| γ Sterilized NE Laboratory Dishes ECO (Sterilized Laboratory Dishes) | Eiken Chemical Co., Ltd. |

**[Table 4]**

| Used Devices | |
|---|---|
| Device Name | Name of Manufacturer |
| Incubator | SANYO Electric Co., Ltd. |
| High Pressure Vapor Sterilizer | Hirayama Manufacturing Corp. |

### Production Example 1

- Ethanol A: 99.5% ethanol composition,
- Ethanol B: 99.8% fermented ethanol produced by Japan Alcohol Corp. under the trade name of Traceable 99 First Grade,
- Ethanol C: 95.2% fermented ethanol under the trade name of Traceable 95 First Grade, and
- Ethanol D: 99.5% synthetic ethanol derived from petroleum produced by Japan Alcohol Corp. under the trade name of 99 degrees synthetic alcohol
were subjected to evaluation of each component by a method in accordance with JAAS001 "Ethanol" in Japan Alcohol Association Standard. The identification limit of 2-propanol was 1 ppm and the lower limits of detecting the other components were 0.01 ppm. The results are shown in Table 5.

**[Table 5]**

| | Lower Limit of Detection (ppm) | Ethanol A | Ethanol B | Ethanol C | Ethanol D |
|---|---|---|---|---|---|
| | | (ppm) | (ppm) | (ppm) | (ppm) |
| Type | | Fermentation | Fermentation | Fermentation | Synthesis |
| 2-Propanol | 1 | Undetected | 2 | 3 | 14 |
| Toluene | 0.01 | 0.07 | Undetected | Undetected | Undetected |
| N-Heptane | 0.01 | 0.04 | Undetected | Undetected | Undetected |
| N-Octane | 0.01 | 0.02 | Undetected | Undetected | Undetected |
| N-Decane | 0.01 | 0.32 | Undetected | Undetected | Undetected |
| Ethylbenzene | 0.01 | 0.30 | Undetected | Undetected | Undetected |

### Example 1

Preparation of Used Culture Mediums and Reagents

### (1) SCDLP

The SCDLP broth culture medium was dissolved in ion exchange water and 9 mL each was dispensed into a 15 mL centrifuge tube, followed by autoclaving.

### (2) Preparation of Sample Solutions

Ethanol A was diluted as a stock solution with PW to have a 15 mass% aqueous ethanol solution to prepare sample solutions.

### (3) Preparation of Bacterial Suspension

(3-1) S. aureus was inoculated on the SCD and cultured at a temperature from 30°C to 35°C for 18 to 24 hours.
(3-2) A colony thus produced was inoculated on 10 mL of SCD-B and cultured at 35°C for 18 to 24 hours to prepare a bacterial suspension.

### Sterilization Effect Test

### (1) Preparation of Bacterial Suspension for Inoculation

The bacterial suspension was diluted with PW and prepared at approximately 10⁷ to 10⁸ CFU/mL as a bacterial suspension for inoculation.

### (2) Inoculation and Action of Bacterial Suspension for Inoculation

(2-1) Respective 10 mL of the sample solutions were dispensed into three 15 mL centrifuge tubes and then 0.1 mL each of the bacterial suspension for inoculation was inoculated and mixed (number of samples n = 3).
(2-2) After 10 mL of a control substance (0% aqueous solution) was dispensed into one 15 mL centrifuge tube, 0.1 mL of the bacterial suspension for inoculation was inoculated and mixed.
(2-3) After acted at room temperature for 10 minutes and 30 minutes, 1 mL each was inoculated on 9 mL of SCDLP and mixed. (neutralization solution)

### (3) Measurement of Viable Cell Count

(3-1) Part of the neutralization solution obtained in (2) above was diluted with SAL to prepare a 10-fold serially diluted solution.
(3-2) On respective two sterilized laboratory dishes, 1 mL each of the neutralization solution and the 10-fold serially diluted solution were inoculated and solidified by adding SCD-A kept warm at 45°C or less.
(3-3) They were cultured at a temperature from 30°C to 35°C for 40 to 48 hours.
(3-4) The number of colonies grown on the two culture media and averaged to be converted to the number of bacteria per 1 mL of the sample solution and the control substance.

### Evaluation

Table 2 shows average values for the number of samples n = 3. The results of the control substance (water only without adding ethanol A, n = 1) are similarly shown in Table 6 as Reference Example 1.

### Comparative Example 1

Evaluation was made under the same conditions as those in Example 1 except for substituting ethanol A with ethanol B. The results are shown in Table 6.

### Comparative Example 2

Evaluation was made under the same conditions as those in Example 1 except for substituting ethanol A with ethanol D. The results are shown in Table 6.

**[Table 6]**

| | Aqueous Ethanol Solution | | Viable Cell Count (CFU/mL) | |
|---|---|---|---|---|
| | Type | Concentration | After 10 Min | After 30 Min |
| Example 1 | Ethanol A | 15% | 246,667 | 170,000 |
| Comparative Example 1 | Ethanol B | 15% | 233,333 | 136,667 |
| Comparative Example 2 | Ethanol D | 15% | 233,333 | 136,667 |
| Reference Example 1 | Water Only | 0% | 240,000 | 240,000 |

From the results in Table 6, it was found that use of the ethanol composition of the present invention caused significant reduction in sterilizability with an ethanol concentration of 15%.

It was thus found that, in the microorganism fermenter, the sterilizability to the microorganism was inhibited with an ethanol concentration of 15% or less, which is an upper limit for production by fermentation, to allow an ethanol culture broth with a higher concentration to be obtained.

Moreover, t it was found that he ethanol composition of the present invention had a reduced sterilization effect relative to the various types of ethanol that were commercially available in the past.

It should be noted that, although not shown in Table 6, similar experiments with concentrations of 30% and 45% were carried out and all types of ethanol resulted in a viable cell count of 0 within 10 minutes, and it was thus found that the ethanol composition of the present invention is particularly suitable for applications at low concentrations.

## Claims

1. A method of producing ethanol by fermenting a carbon source by a microorganism using a eubacterium or an archaebacterium, wherein a 2-propanol concentration in a microorganism fermenter is less than 2 ppm.

2. The method of producing ethanol according to Claim 1, wherein the carbon source contains a biomass resource.

3. The method of producing ethanol according to Claim 2, wherein the biomass resource is an inedible material.

4. The method of producing ethanol according to Claim 2 or 3, wherein the biomass resource is a waste.

5. The method of producing ethanol according to any one of Claims 1 through 4, wherein the microorganism is a eubacterium.

6. The method of producing ethanol according to any one of Claims 1 through 5, wherein an ethanol concentration in the microorganism fermenter is 0.1 mass% or more and 15 mass% or less.

7. Ethanol produced by the method according to any one of Claims 1 through 6.

8. An ethanol composition having a 2-propanol concentration of less than 2 ppm.

9. An ethanol composition comprising 0.001 ppm or more and 1000 ppm or less of a saturated hydrocarbon compound with a carbon number from 6 to 14.

10. The ethanol composition according to Claim 9, comprising 50 mass% or more of ethanol.

11. The ethanol composition according to any one of Claims 8 through 10, further comprising from 0.001 ppm to 800 ppm of an aromatic hydrocarbon compound with a carbon number from 6 to 14.

12. The ethanol composition according to any one of Claims 8 through 11, wherein the saturated hydrocarbon compound is one or more selected from the group consisting of heptane, octane, and decane.

13. An ethanol composition comprising from 0.001 ppm to 100 ppm of an aromatic compound with a carbon number from 6 to 14.

14. The ethanol composition according to Claim 13, wherein the aromatic compound is one or more selected from the group consisting of toluene, benzene, ethylbenzene, xylene, and naphthalene.

15. The ethanol composition according to Claim 8, 13, or 14, further comprising from 0.001 ppm to 1000 ppm of a saturated hydrocarbon compound with a carbon number from 6 to 14.

16. The ethanol composition according to any one of Claims 8 through 15, comprising 50 mass% or more of ethanol.

17. An ethanol composition comprising from 0.001 ppm to 1000 ppm of n-decane.

18. The ethanol composition according to Claim 17, comprising from 0.001 ppm to 800 ppm of ethylbenzene.

19. An ethanol composition comprising from 0.001 ppm to 100 ppm of ethylbenzene.

20. The ethanol composition according to Claim 19, comprising from 0.001 ppm to 1000 ppm of n-decane.

21. A soil disinfection method, comprising:
diluting the ethanol composition according to any one of Claims 8 through 20 with water; and
distributing an aqueous ethanol solution with an ethanol concentration after dilution from 0.1 to 15 mass %.
